# FASCICULE DE BREVET EUROPEEN

(11) **EP 0 571 299 B1**
(45) Date de publication et mention de la délivrance du brevet: **19.03.1997**
(21) Numéro de dépôt: 93420185.6
(22) Date de dépôt: 07.05.1993
(51) Int. Cl.: C07C 51/09, C07C 27/02

(54) **Procédé d'hydrolyse de dicarboxylates d'alkyle**
Verfahren zur Hydrolysierung von Alkyldicarboxylaten
Process for hydrolysing alkyl dicarboxylates

(30) Priorité: 20.05.1992 FR 9206392
(43) Date de publication de la demande: 24.11.1993
(73) Titulaire: RHONE-POULENC FIBER & RESIN INTERMEDIATES, 92405 Courbevoie (FR)
(72) Inventeur: Berrod, Gérard, F-69100 Villeurbanne (FR); Grillon, Eric, F-58500 Armes (FR); Klinger, Francois, F-Chaponost (FR)
(74) Mandataire: Vignally, Noel

(56) Documents cités:
- EP-A- 0 134 099
- DE-B- 1 155 780
- US-A- 2 719 166

## Description

La présente invention concerne l'hydrolyse de dicarboxylate d'alkyle inférieur, c'est-à-dire d'alkyle ayant 1 à 4 atomes de carbone, en acide dicarboxylique.

Très généralement l'hydrolyse des esters s'effectue en milieu homogène soit à l'aide d'un acide, soit à l'aide d'une base.

L'utilisation d'une base conduit à la formation de sels métalliques avec les inconvénients évidents que cela suppose.

L'hydrolyse acide des adipates présente le problème du caractère biphasique du milieu, dû à la très faible miscibilité de l'eau et de l'adipate. C'est pourquoi il a été préconisé d'utiliser des mélanges de solvants (voir par exemple JOURNAL OF INDIAN CHEMICAL SOCIETY, 48(9) pages 811-12), ce qui complique notablement la séparation et la purification ultérieures de l'acide adipique obtenu. En outre l'utilisation d'un acide liquide entraîne d'importants problèmes de corrosion de l'appareillage.

Il a également été préconisé d'opérer l'hydrolyse des adipates en milieu hétérogène sur des résines acides.

Ainsi un article publié dans SYNTHETIC COMMUNICATIONS 19, pages 627-631 (1989) décrit l'hydrolyse de l'adipate de diméthyle dans une suspension aqueuse de résine DOWEX-50, au reflux. Ce type de réaction en suspension ne résoud pas le problème du déplacement de l'équilibre de la réaction par l'élimination du méthanol formé.

Le brevet EP-A-0 056 489 propose une solution à ce problème, consistant en un procédé continu d'hydrolyse par l'eau des adipates d'alkyle de 1 à 4 atomes de carbone, à température élevée et en présence de résines échangeuses d'ions fortement acides ; la caractéristique principale de ce procédé consiste à prélever une partie du mélange réactionnel eau/adipate sur plusieurs plateaux de la colonne, au-dessous de la zone d'arrivée de ce mélange dans la colonne, la partie ainsi prélevée est ensuite passée sur un échangeur d'ion fortement acide, puis est recyclée. On obtient ainsi de l'acide adipique au bas de la colonne alors que les mélanges alcanol/eau sont prélevés en tête de colonne.

Ce procédé nécessite l'adjonction de plusieurs circuits annexes à la colonne pour réaliser la réaction d'hydrolyse proprement dite et sa mise en oeuvre est donc complexe.

Le brevet DE-B-1 150 780 décrit un procédé de mise en oeuvre de réactions catalysées par des acides ou des bases et dédit plus spécifiquement l'hydrolyse du succinate de diméthyle par introduction sur une colonne comportant un catalyseur à base de résine échangeuse d'ions à groupes acide sulfonique tandis que de l'eau est chauffée à ébullition dans un ballon situé sous la colonne. La réaction de saponification s'effectue dans la colonne et d'une part le méthanol formé distille et est récupéré en tête de colonne et d'autre part le mélange d'acide succinique et d'eau est récupéré dans le ballon en bas de la colonne.

Le procédé ne se prête pas à une marche en continu

La présente invention se propose de résoudre le problème de l'obtention d'acide dicarboxylique par hydrolyse de dicarboxylates d'alkyles de 1 a 4 atomes de carbone par la mise en oeuvre d'un procédé simplifié, mais très efficace, dans lequel la résine acide utilisée répond à une série de caractéristiques judicieusement sélectionnées.

Ce procédé permet de réaliser à la fois l'hydrolyse du dicarboxylate et la séparation de l'alcanol formé.

Plus spécifiquement, l'invention consiste en un procédé de préparation d'acide dicarboxylique par hydrolyse en phase liquide d'un dicarboxylate aliphatique d'alkyle en C₁-C₄, caractérisé en ce que l'on soumet un mélange dudit dicarboxylate d'alkyle et d'eau à une distillation réactive, par introduction dudit mélange dans la partie médiane d'une colonne contenant une résine de type gel, à groupes acide sulfonique, maintenue en place à l'aide d'un support et présentant les caractéristiques suivantes :
- un squelette à base de styrène et de divinylbenzène, le taux de divinylbenzène en poids par rapport au poids total dudit squelette étant compris entre 1 % et 12 % et de préférence entre 2 % et 5 %,
- une capacité d'échange de 3 à 6,5 milliéquivalents H⁺/gramme de résine sèche,
et par distillation continue de l'alcanol formé par la réaction d'hydrolyse.

Dans le cadre du présent procédé, on utilisera de préférence une résine de type gel à groupes acide sulfonique ayant les caractéristiques suivantes :
- un squelette à base de styrène et de divinylbenzène, le taux de divinylbenzène en poids par rapport au poids total dudit squelette étant compris entre 1 % et 12 % et de préférence entre 2 % et 5 %,
- une capacité d'échange de 5 à 6,5 milliéquivalents H⁺/gramme de résine sèche,
- une granulométrie moyenne comprise entre 100 et 2000 micromètres et de préférence entre 200 et 1200 micromètres.

De manière générale, la colonne qui peut être utilisée comprend un tronçon inférieur dans lequel est disposée la résine et où s'effectue l'hydrolyse et un tronçon supérieur, comportant éventuellement un garnissage classique, dans lequel s'effectue la purification du méthanol.

Les résines de type gel à groupes acide sulfonique sont des résines connues en elles-mêmes.

On choisira parmi les résines-gels commerciales celles qui répondent aux caractéristiques indiquées précédemment.

La résine-gel peut être mise en oeuvre dans la colonne selon des techniques variées. Elle peut être maintenue dans une structure métallique, textile, céramique ou plastique assurant un échange liquide-solide et liquide-vapeur efficace, la structure contenant la résine étant disposée dans la colonne.

Elle peut également être déposée ou enrobée sur une telle structure.

La résine-gel peut également être placée sur des plateaux capacitifs.

Ces mises en oeuvre ne sont pas limitatives.

Les dicarboxylates aliphatiques d'alkyle de C₁ à C₄ qui peuvent être hydrolysés dans le procédé de l'invention sont essentiellement les esters d'alkyle de C₁ à C₄ d'acides dicarboxyliques aliphatiques ayant 3 à 6 atomes de carbone, tels que par exemple les esters de méthyle, d'éthyle, de n-propyle des acides succinique, éthyl-succinique,glutarique, méthyl-glutarique, adipique, hexène-2-dioïque ou hexène-3-dioïque.

Parmi ces esters, l'adipate de diméthyle, le méthyl-glutarate de diméthyle, l'éthyl-succinate de diméthyle et les hexène-dioates de diméthyle sont les plus importants et l'adipate de diméthyle est le composé dont l'hydrolyse est encore plus particulièrement importante.

Ces esters et plus spécifiquement l'adipate de diméthyle peuvent être mis en oeuvre isolément ou en un mélange de plusieurs d'entre eux.

Dans ce qui suit, il sera question par commodité de l'hydrolyse de l'adipate de diméthyle, mais cela sera transposable à un autre dicarboxylate d'alkyle tel que défini précédemment.

L'adipate de diméthyle et l'eau sont introduits dans la colonne dans la partie médiane de celle-ci, au-dessus du tronçon où se trouve la résine-gel.

La quantité d'eau est telle que le rapport molaire eau/adipate de diméthyle est au moins égal à 2.

De préférence, la quantité d'eau sera en excès par rapport à la quantité stoechiométrique, c'est-à-dire que le rapport molaire eau/adipate de diméthyle sera supérieur à 2, généralement compris entre 5 et 60.

L'adipate de diméthyle et l'eau peuvent être introduits séparément ou en mélange.

L'hydrolyse de l'adipate de diméthyle s'effectue sur la résine-gel et l'on recueille d'une part, à l'extrémité supérieure de la colonne,le méthanol formé et d'autre part, à l'extrémité inférieure de la colonne,l'acide adipique, l'excès d'eau, l'éventuel adipate de monométhyle et l'éventuelle quantité d'adipate de diméthyle non hydrolysés.

La température dans la colonne se situe généralement entre 80°C et 150°C dans la partie où s'effectue la réaction d'hydrolyse et préférentiellement entre 90°C et 130°C.

L'acide adipique préparé par le procédé selon l'invention est séparé de l'eau et des autres composés minoritaires qu'il peut comprendre par les méthodes usuelles de séparation utilisées en chimie, par exemple par cristallisation, notamment par cristallisation sous vide.

Les eaux-mères contenant généralement entre 2% et 10% d'acide adipique et les différentes impuretés ou sous-produits de la réaction, peuvent avantageusement être recyclées dans la colonne d'hydrolyse.

Dans les exemples illustratifs de l'invention qui suivent, la productivité de l'hydrolyse sera exprimée en kg d'acide adipique par kg de résine-gel sèche par heure.

### EXEMPLES

L'appareillage utilisé pour les différents exemples et essais comparatifs est constitué d'une colonne de 4 cm de diamètre intérieur, composée de deux tronçons:
- un tronçon inférieur de 45 cm de longueur dans lequel est disposée la résine, appelé ci-après tronçon réactif,
- un tronçon supérieur de 150 cm de longueur, comportant un garnissage classique permettant la séparation eau-méthanol.

Cette colonne est équipée d'un bouilleur à sa base, d'un condenseur en tête et d'un dispositif de reflux ; l'alimentation s'effectue au sommet du tronçon réactif gràce à deux pompes: l'une pour l'eau, l'autre pour l'adipate de diméthyle.

Le méthanol formé est obtenu en tête de colonne tandis que le mélange eau-acide adipique est soutiré en pied.

La résine est disposée dans le tronçon réactif de la colonne selon deux types de conditionnement.

### 1) Conditionnement a)

La résine est placée à l'intérieur de quatre fuseaux dont les parois sont constituées d'une toile métallique ayant une maille d'environ 500 µm, inférieure au diamètre des billes de la résine.

Ces fuseaux, longs de 45 cm, sont fermés à leurs extrémités. Ils sont maintenus côte à côte par un tissu métallique, afin d'assurer la cohésion de l'ensemble et favoriser les échanges liquide-vapeur, ainsi que la redistribution du liquide vers les fuseaux contenant la résine.

La quantité de résine représente ainsi 26 % du volume du tronçon réactif de la colonne. Le nombre d'étages théoriques de ce tronçon est de 4. Chaque étage théorique comporte 36,25 g de résine humide (à 65 % ou 50 % d'humidité selon la résine mise en oeuvre).

### 2) Conditionnement b)

La résine est disposée à l'intérieur d'un sandwich constitué de deux couches de toile métallique ayant une maille d'environ 500 µm. Ce sandwich, contenant une couche mince de résine a été plié "en accordéon" avec une amplitude des plis telle qu'il puisse garnir l'ensemble de la section du tronçon de colonne.

Chaque sandwich ainsi plié a une hauteur de 15 cm. Trois sont donc nécessaires pour garnir les 45 cm du tronçon réactif de la colonne. Chaque sandwich, à l'intérieur de la colonne, est orienté perpendiculairement par rapport au précédent, afin d'éviter les passages préférentiels hors des zones chargées en résine.

La quantité de résine représente ainsi 32 % du volume du tronçon réactif de la colonne. Le nombre d'étages théoriques de ce tronçon est de 4. Chaque étage théorique comporte 45 g de résine humide (à 65 % ou 50 % d'humidité selon la résine mise en oeuvre).

Ces deux types de conditionnement de la résine constituent un garnissage réactif permettant de catalyser une réaction et de favoriser les échanges liquide-vapeur pour effectuer une séparation par distillation.

### EXEMPLES 1 ET 2

Ces deux exemples sont effectués avec une résine-gel A à groupes acide sulfonique, qui se présente sous forme de billes de 1000 µm de diamètre et qui possède un taux de divinylbenzène en poids par rapport au poids total du squelette à base de styrène et de divinylbenzène (ou taux de réticulation) de 4 % , une capacité d'échange de 5,4 milliéquivalents (meq) H⁺/g et un taux d'humidité de 65 % environ.

Ces 2 exemples sont réalisés avec le conditionnement de la résine de type a) décrit précédemment.

Les débits d'alimentation sont les suivants :
- - adipate de diméthyle (ADM) :: 188g/h (exemple 1)
100g/h (exemple 2)
- - eau:: 295g/h (exemple 1)
155g/h (exemple 2)

La pression de travail est égale à la pression atmosphérique.

En régime permanent, la température en tête de colonne est de 65°C (c'est-à-dire la température d'ébullition du méthanol pur sous pression atmosphérique). La température en pied est de 101°C.

Des analyses par CLHP (chromatographie liquide haute performance) permettent de calculer le taux de transformation (TT) de l'ADM, le rendement (RR) en acide adipique (AA) exprimé en mol % par rapport à l'ADM chargé et la productivité de la réaction exprimée en kg d'AA par kg de résine sèche par heure.

Ces résultats ainsi que diverses indications sont rassemblés dans le tableau ci-après.

### EXEMPLE 3 ET ESSAI COMPARATIF 1

Ces deux essais sont effectués :
- l'un (exemple 3) avec la résine-gel décrite pour les exemples 1 et 2 (résine A)
- l'autre (essai comparatif 1) avec une résine macroporeuse B à squelette polystyrène/divinylbenzène et à groupes acide sulfonique qui ne répond pas à toutes les caractéristiques revendiquées : taux de réticulation de 16 %, capacité d'échange de 5,4 meq H⁺/g de résine sèche, billes de 1000 µm de diamètre, taux d'humidité de 50 % environ, les deux résines étant mises en oeuvre avec le conditionnement de type b) décrit précédemment.

Les conditions de mise en oeuvre de ces essais sont les mêmes que pour les exemples 1 et 2, à l'exception des débits d'alimentation qui sont les suivants:
- ADM: 152 g/h
- eau: 295 g/h

Les résultats obtenus ainsi que diverses indications sont rassemblés dans le tableau ci-après.

### ESSAI COMPARATIF 2

Cet essai a été réalisé avec la résine A, en quantité égale à celle utilisée dans l'exemple 3, mais en la disposant dans le bouilleur et non dans la colonne. Il ne s'agit donc pas d'une distillation réactive comme dans le procédé de l'invention, mais de l'utilisation d'un réacteur agité continu, surmonté d'une colonne pour éliminer le méthanol formé.

Les débits d'alimentation et les conditions opératoires sont ceux de l'exemple 3.

Les résultats obtenus ainsi que diverses indications sont rassemblés dans le tableau ci-après.

### EXEMPLE 4

Cet exemple a été réalisé avec la résine A, disposée selon le conditionnement de type b) décrit précédemment.

Afin d'obtenir un rendement en AA plus élevé que dans les exemples précédents, la colonne utilisée comporte un tronçon réactif de 99 cm avec pratiquement 9 étages théoriques.

Les débits d'alimentation et les conditions opératoires sont ceux de l'exemple 3.

Les résultats obtenus ainsi que diverses indications sont rassemblés dans le tableau ci-après.

### ESSAI COMPARATIF 3

Cet essai a été réalisé avec la résine B, disposée selon le conditionnement de type b) décrit précédemment.

Afin d'obtenir un rendement en AA plus élevé que dans les exemples précédents, la colonne utilisée comporte un tronçon réactif de 340 cm avec 30 étages théoriques.

Les débits d'alimentation et les conditions opératoires sont ceux de l'exemple 3.

Les résultats obtenus ainsi que diverses indications sont rassemblés dans le tableau ci-après.

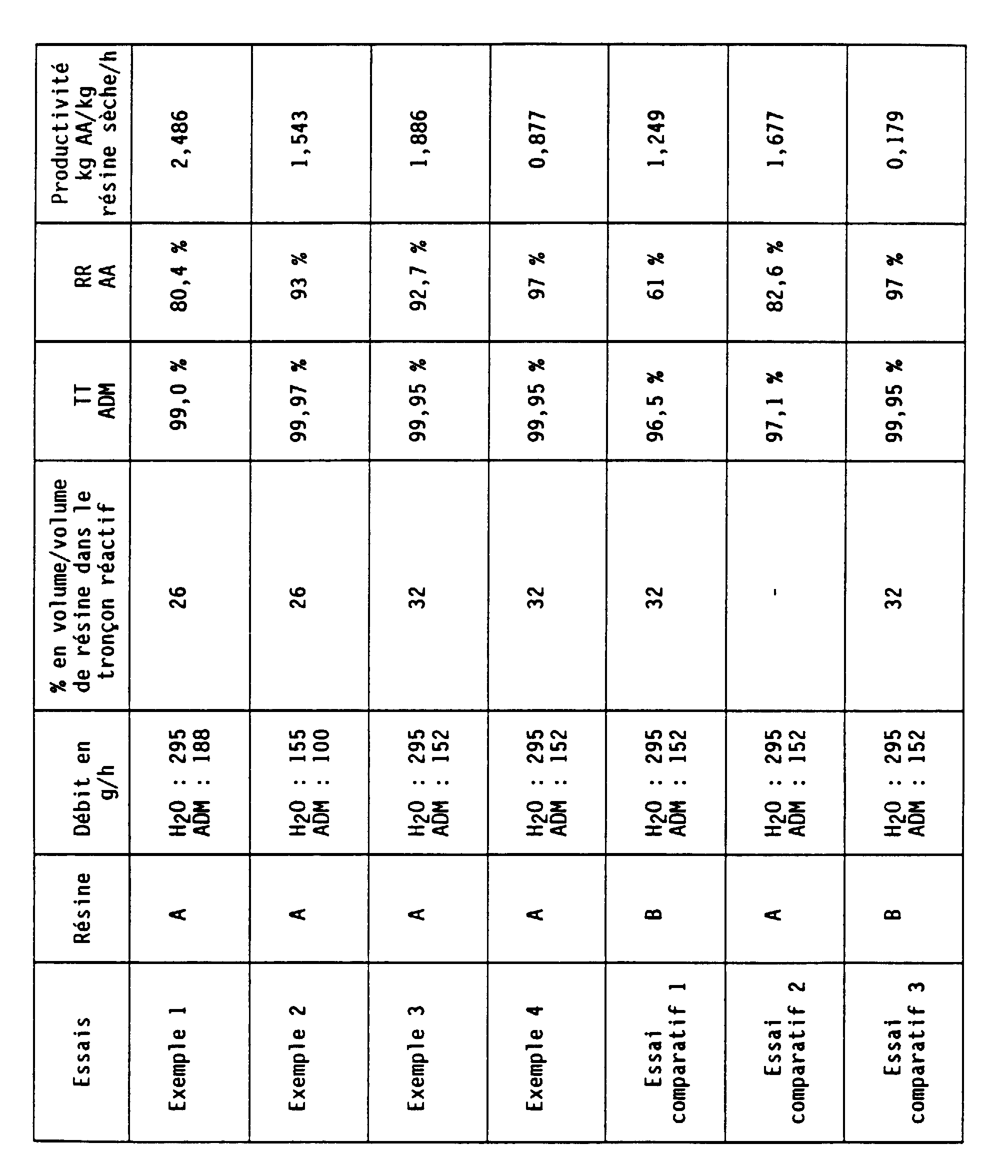

## Revendications

1. Procédé de préparation d'acide dicarboxylique par hydrolyse en phase liquide de dicarboxylate aliphatique d'alkyle en C₁-C₄, caractérisé en ce que l'on soumet un mélange dudit dicarboxylate d'alkyle et d'eau à une distillation réactive, par introduction dudit mélange dans la partie médiane d'une colonne contenant une résine de type gel à groupes acide sulfonique, maintenue en place à l'aide d'un support et présentant les caractéristiques suivantes :
- un squelette à base de styrène et de divinylbenzène, le taux de divinylbenzène en poids par rapport au poids total dudit squelette étant compris entre 1 % et 12 % et de préférence entre 2 % et 5 %,
- une capacité d'échange de 3 à 6,5 milliéquivalents H⁺/gramme de résine sèche,
et par distillation continue de l'alcanol formé par la réaction d'hydrolyse.

2. Procédé selon la revendication 1, caractérisé en ce que l'on utilise une résine de type gel à groupes acide sulfonique ayant les caractéristiques suivantes:
- un squelette à base de styrène et de divinylbenzène, le taux de divinylbenzène en poids par rapport au poids total dudit squelette étant compris entre 1 % et 12 % et de préférence entre 2 % et 5 %,
- une capacité d'échange de 5 à 6,5 milliéquivalents H⁺/gramme, de résine sèche,
- une granulométrie moyenne comprise entre 100 et 2000 micromètres et de préférence entre 200 et 1200 micromètres.

3. Procédé selon l'un des revendications 1 ou 2, caractérisé en ce que les dicarboxylates aliphatiques d'alkyle de C₁ à C₄ sont les esters d'alkyle de C₁ à C₄ d'acides dicarboxyliques aliphatiques ayant 3 à 6 atomes de carbone tels que les esters de méthyle, d'éthyle, de n-propyle des acides succinique, éthyl-succinique, glutarique, méthyl-glutarique, adipique, hexène-2-dioïque , hexène-3-dioïque et leurs mélanges.

4. Procédé selon l'une des revendications 1 ou 2, caractérisé en ce que le dicarboxylate aliphatique d'alkyle de C₁ à C₄ est choisi parmi l'adipate de diméthyle, le méthyl-glutarate de diméthyle, l'éthyl-succinate de diméthyle, les hexène-dioates de diméthyle et leurs mélanges et est de préférence l'adipate de diméthyle.

5. Procédé selon l'une des revendications 1 à 4, caractérisé en ce que l'adipate de diméthyle et l'eau sont introduits dans la colonne dans la partie médiane de celle-ci, au dessus du tronçon où se trouve la résine-gel,la quantité d'eau étant telle que le rapport molaire eau/adipate de diméthyle est au moins égal à 2, de préférence supérieur à 2 et généralement compris entre 5 et 60.

6. Procédé selon l'une des revendications 1 à 5, caractérisé en ce que l'hydrolyse de l'adipate de diméthyle s'effectue sur la résine-gel et que l'on recueille d'une part, à l'extrémité supérieure de la colonne,le méthanol formé et d'autre part, à l'extrémité inférieure de la colonne,l'acide adipique, l'excès d'eau, l'éventuel adipate de monométhyle et l'éventuelle quantité d'adipate de diméthyle non hydrolysés.

7. Procédé selon l'une des revendications 1 à 6, caractérisé en ce que la température dans la colonne se situe entre 80°C et 150°C dans la partie ou s'effectue la réaction d'hydrolyse et plus préférentiellement entre 90°C et 130°C.

## Patentansprüche

1. Verfahren zur Herstellung von Dicarbonsäuren durch Hydrolyse aliphatischer Alkyl (C₁-C₄)-dicarboxylate in flüssiger Phase, dadurch gekennzeichnet, daß eine Mischung des besagten Alkyldicarboxylats mit Wasser einer reaktiven Destillation unterworfen wird, indem diese Mischung in den mittleren Teil einer Säule eingebracht wird, die mit einem Sulfonsäuregruppen enthaltenden Ionenaustauscher-Gel bestückt ist, welches mit Hilfe einer Halterung fixiert wird und folgende Eigenschaften besitzt:
- ein von Styrol und Divinylbenzol abgeleitetes Grundgerüst, wobei der Gewichtsanteil des Divinylbenzols an der Gesamtmasse des besagten Grundgerüsts zwischen 1 und 12%, vorzugsweise zwischen 2 und 5% beträgt,
- eine Ionenaustauschkapazität zwischen 3 und 6,5 Milliäquivalenten H⁺/Gramm trockenem Ionenaustauscher-Gel,
und indem der bei der Hydrolysereaktion gebildete Alkylalkohol kontinuierlich abdestilliert wird.

2. Verfahren nach Anspruch 1, dadurch gekennzeichnet, daß ein Sulfonsäuregruppen enthaltendes Ionenaustauscher-Gel eingesetzt wird, das folgende Eigenschaften besitzt:
- ein von Styrol und Divinylbenzol abgeleitetes Grundgerüst, wobei der Gewichtsanteil des Divinylbenzols an der Gesamtmasse des besagten Grundgerüsts zwischen 1 und 12%, vorzugsweise zwischen 2 und 5% beträgt,
- eine Ionenaustauschkapazität zwischen 5 und 6,5 Milliäquivalenten H⁺/Gramm trockenem Ionenaustauscher-Gel,
- eine mittlere Korngröße zwischen 100 und 2000 Mikrometern, vorzugsweise zwischen 200 und 1200 Mikrometern.

3. Verfahren nach einem der Ansprüche 1 und 2, dadurch gekennzeichnet, daß die aliphatischen Alkyl (C₁-C₄)-dicarboxylate Alkyl (C₁-C₄)-ester aliphatischer Dicarbonsäuren mit 3 bis 6 Kohlenstoffatomen sind, so zum Beispiel die Methyl-, Ethyl- und n-Propylester der Bernsteinsäure, der Ethylbernsteinsäure, der Glutarsäure, der Methylglutarsäure, der Adipinsäure, der 2-Hexendisäure, der 3-Hexendisäure und ihrer Mischungen.

4. Verfahren nach einem der Ansprüche 1 und 2, dadurch gekennzeichnet, daß das aliphatische Alkyl (C₁-C₄)-dicarboxylat aus Adipinsäuredimethylester, Methylglutarsäuredimethylester, Ethylbernsteinsäuredimethylester, 2-Hexensäure- bzw. 3-Hexensäuredimethylester und ihren Mischungen ausgewählt wird und vorzugsweise Adipinsäuredimethylester ist.

5. Verfahren nach einem der Ansprüche 1 bis 4, dadurch gekennzeichnet, daß der Adipinsäuredimethylester und das Wasser in den mittleren Teil der Säule eingebracht werden, oberhalb des Abschnitts, in dem sich das Ionenaustauscher-Gel befindet, wobei die Menge an Wasser so gewählt wird, daß das molare Verhältnis Wasser/Adipinsäuredimethylester mindestens 2 beträgt, vorzugsweise größer 2 ist und im allgemeinen zwischen 5 und 60 liegt.

6. Verfahren nach einem der Ansprüche 1 bis 5, dadurch gekennzeichnet, daß die Hydrolyse des Adipinsäuredimethylesters auf dem Ionenaustauscher-Gel stattfindet und daß - zum einen - am Kopf der Säule das gebildete Methanol und - zum anderen - am Fuß der Säule die Adipinsäure, das überschüssige Wasser sowie gegebenenfalls Adipinsäuremonomethylester und gegebenenfalls nicht hydrolysierter Adipinsäuredimethylester entnommen werden.

7. Verfahren nach einem der Ansprüche 1 bis 6, dadurch gekennzeichnet, daß die Temperatur in dem Teil der Säule, in dem die Hydrolysereaktion stattfindet, zwischen 80 und 150°C beträgt, vorzugsweise zwischen 90 und 130°C.

## Claims

1. Process for the preparation of a dicarboxylic acid by hydrolysis in the liquid phase of a C₁-C₄ alkyl aliphatic dicarboxylate, characterized in that a mixture of the said alkyl dicarboxylate and water is subjected to a reactive distillation by introduction of the said mixture into the middle part of a column containing a gel-type resin containing sulphonic acid groups, maintained in place using a support and having the following characteristics:
- a skeleton based on styrene and divinylbenzene, the level of divinylbenzene by weight with respect to the total weight of the said skeleton being between 1 % and 12 % and preferably between 2 % and 5 %,
- an exchange capacity of 3 to 6.5 milliequivalents H⁺/gram of dry resin,
and by continuous distillation of the alcohol formed by the hydrolysis reaction.

2. Process according to claim 1, characterized in that a gel-type resin containing sulphonic acid groups and having the following characteristics is used:
- a skeleton based on styrene and divinylbenzene, the level of divinylbenzene by weight with respect to the total weight of the said skeleton being between 1 % and 12 % and preferab y between 2 % and 5 %,
- an exchange capacity of 5 to 6.5 milliequivalents H⁺/gram of dry resin,
- a mean particle size of between 100 and 2000 micrometres and preferably between 200 and 1200 micrometres.

3. Process according to either of claims 1 or 2, characterized in that the C₁ to C₄ alkyl aliphatic dicarboxylates are C₁ to C₄ alkyl esters of aliphatic dicarboxylic acids having 3 to 6 carbon atoms such as the methyl, ethyl or n-propyl esters of succinic, ethylsuccinic, glutaric, methylglutaric, adipic, 2-hexenedioic and 3-hexenedioic acids and their mixtures.

4. Process according to either of claims 1 or 2, characterized in that the C₁ to C₄ alkyl aliphatic dicarboxylate is chosen from dimethyl adipate, dimethyl methylglutarate, dimethyl ethylsuccinate, the dimethyl hexenedioates and their mixtures and is preferably dimethyl adipate.

5. Process according to one of claims 1 to 4, characterized in that dimethyl adipate and water are introduced into the column in the middle part of the latter, above the section where the resin/gel is situated, the amount of water being such that the water/dimethyl adipate molar ratio is at least equal to 2, preferably greater than 2 and generally between 5 and 60.

6. Process according to one of claims 1 to 5, characterized in that hydrolysis of dimethyl adipate is carried out on the resin/gel and in that there is recovered, on the one hand, at the upper end of the column, the methanol formed and, on the other hand, at the lower end of the column, the adipic acid, the excess water, the possible nonhydrolysed monomethyl adipate and the possible amount of nonhydrolysed dimethyl adipate.

7. Process according to one of claims 1 to 6, characterized in that the temperature in the column is between 80°C and 150°C in the part where the hydrolysis reaction is carried out and more preferentially between 90°C and 130°C.
